# EUROPEAN PATENT APPLICATION

(11) **EP 1 985 268 A1**
(43) Date of publication of application: **29.10.2008**
(21) Application number: 07251766.7
(22) Date of filing: 26.04.2007
(51) Int. Cl.: A61F 7/02

(54) **Therapeutic treatment device for cold sores and the like**

(71) Applicant: The Jenex Corporation, Burlington, ON L7L 5K6 (CA)
(72) Inventor: Felice, Donald F., Alliance, Ohio 44601 (US); Jenkins, Michael A., Burlington, Ontario L7P 3H6 (CA)
(74) Representative: Makovski, Priscilla Mary

(57) **Abstract**

A therapeutic treatment device (10) for treating cold sores and the like includes a housing (12) configured to be hand held, and having an annular pad (20) at one end thereof configured to engage an area of the body of the user. A heat source (24) is maintained adjacent the pad. A controller (36), in the form of a programmable chip or the like, allows for the heat source (24) to be energised for a particular period of time, shutting off automatically. After a predetermined quiescent period of time has expired, the device (10) signals the user that the heat source (24) can be energised again, with the controller (36) then maintaining the activation of the heat source (24) for a second preselected period of time. The cycle continues until the treatment is complete. A treatment actuator (38) communicates with the controller (36) to enable the controller to process through the treatment sequence. The treatment actuator (38) may be specifically tailored to any of various treatments, including the treatment for a cold sore, an insect bite, a bee sting, a spider bite, or the like.

## Description

### TECHNICAL FIELD

The invention herein resides in the art of therapeutic devices and, more particularly, to such devices for use in the treatment of cold sores, insect bites, bee stings, spider bites and the like. Particularly, the invention relates to a hand held therapeutic device that provides heat to a specified area of the body for particular periods of time and at particular intervals in order to provide for the prevention and relief of the symptoms of cold sores, and to hasten recovery from other maladies.

### BACKGROUND OF THE INVENTION

The invention herein will be described with particular regard to its application to the treatment of cold sores. However, the concept of the invention may also be extendable and applicable to the treatment of other viral or bacterially caused conditions. Additionally, the concept of the invention extends to insect bites, bee stings, spider bites, and similar maladies for which it has been found that the localized application of heat for particular spaced durations of time hastens recovery. Applicants have particularly found that the application of heat to the cold sore area, particularly before visible manifestation, can prevent the visible manifestation or, at least, shorten its duration.

Cold sores are common sources of personal frustration and embarrassment. They are commonly triggered by infections, exposure to the sun, fever, stress, fatigue, hormonal changes, trauma or injury, pregnancy, or exposure to excessive temperatures.

An individual may sense that a cold sore is starting by feeling a tingling, prickling, itching or burning sensation about the lips at the site of the cold sore, well before the cold sore is visibly detectable. This is called the prodrome or prodromal stage. This stage generally precedes the eruption of a cold sore lesion by about 24 hours. At that time, the area will become red and a fluid-filled blister will form. This blister will weep with a clear fluid and then subsequently form a scab. The duration of time from the initial eruption of the cold sore lesion to the time at which the scab falls off and the healing is complete is on the order of 10-14 days.

Applicants have found that the localized application of heat to the cold sore region in the prodomal stage can prevent the cold sore lesion from forming and, if applied later in the stage, or if ineffective to preclude the formation of the lesion, such application of localized heat can shorten the duration of the lesion by a considerable time. As mentioned above, similar effects have been known to take place with insect bites, bee stings, and the like.

The prior art has recognized that the application of heat to the localized affected region of a bee sting or insect bite has curative results. The art has addressed this concept in a somewhat rudimentary fashion in prior art patent 4,944,297. The prior art, however, has failed to recognize that cold sores may be effectively treated by the application of heat to the localized affected region for particular periods of time and at a particular frequency or temporal spacing. The prior art has similarly failed to recognize that the heat treatment of bee stings and insect bites may be significantly improved by the time-regulated application of heat.

The prior art is unaware of the benefits to be derived from the localized application of heat to cold sores in general, and the time-regulated application of heat to cold sores and other maladies in particular. Specifically, there is a need for a mechanism for delivering heat to an affected area in an effective manner. Particularly, there is a need in the art for a hand held device that effectively walks the user through the required application of heat to the localized area, by signaling the user when each application should begin and when it ends, and when a subsequent application is appropriate. There is a need in the art for a device for providing such a user friendly approach to treatment, and which may be tailored to the treatment of various maladies requiring different durations and spacing of the heat application.

### DISCLOSURE OF THE INVENTION

In light of the foregoing, it is a first aspect of the invention to provide a therapeutic treatment device for cold sores and the like, which allows for ease of application of heat to a localized area.

Another aspect of the invention is the presentation of a therapeutic treatment device for cold sores and the like that is user friendly and instructive in walking the user through the heat application and the sequencing and durations thereof.

Still another aspect of the invention is the provision of a therapeutic treatment device for cold sores and the like that can be tailored for the treatment of any of various incidents requiring the application of heat at different time intervals for different durations.

Yet a further aspect of the invention is the provision of a therapeutic device for cold sores and the like that is adaptable in use for multiple purposes.

Yet another aspect of the invention is the provision of a therapeutic treatment device for cold sores and the like, which is replenishable and reusable, having a cost incident thereto that is commensurate with the aggregate use of the device itself.

The foregoing and other aspects of the invention which will become apparent as the detailed description proceeds are achieved by a therapeutic treatment device, comprising: a housing configured to be hand held; a pad on said housing configured to engage an area on the body of a user; a heat source adjacent said pad; and a controller connected to said heat source, said controller enabling said heat source for determined periods of time at predetermined intervals.

Other aspects of the invention which will become apparent herein are attained by an actuator for a therapeutic treatment device having a housing containing a heat source and a controller connected to said heat source, said controller enabling said heat source for predetermined periods of time at predetermined intervals, the actuator comprising: a clip configured for removable receipt by the housing; and a module carried by said clip, said module configured for communicative interconnection with the controller.

### DESCRIPTION OF DRAWING

For a complete understanding of the objects, techniques and structure of the invention, reference should be made to the following detailed description and accompanying drawing wherein:
Fig. 1 is a perspective view of the therapeutic treatment device of the invention;
Fig. 2 is a perspective view of the actuator clip adapted to enable the controller of the device of Fig. 1; and
Fig. 3 is a schematic block diagram of the circuitry of the invention configured to achieve the beneficial aspects hereof.

### BEST MODE FOR CARRYING OUT THE INVENTION

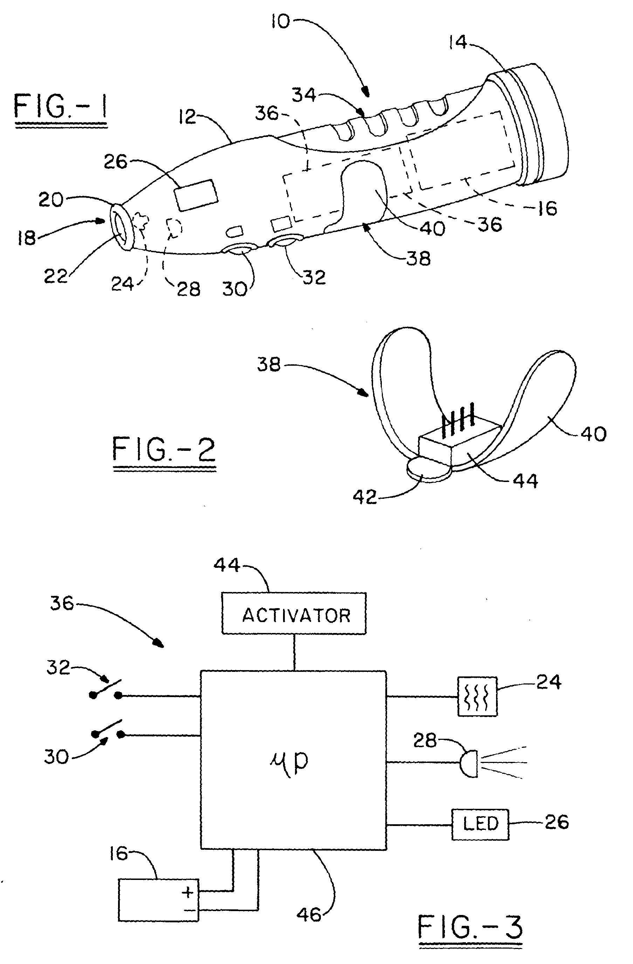

Referring now to the drawing and more particularly Fig. 1, it can be seen that a thermal treatment device made in accordance with the invention is designated generally by the numeral 10. The device 10 includes a housing 12 having an end cap 14, which is removable therefrom and securable thereto by means of screws, a catch and latch mechanism, or the like. A battery 16 is received within the housing 12 near the end receiving the end cap 14, the battery 16 being provided for powering the thermal treatment device 10 as will become apparent herein.

At the end of the device 10, opposite that of the end cap 14, is an applicator tip 18, configured to contact the affected area of skin of the user. The applicator tip 18 includes an annular pad 20 encircling a recessed screen or mesh 22. The screen or mesh 22 prevents a light bulb 24, or other appropriate heat generating device or heat element, from coming into direct contact with the skin of the user when the annular pad 20 is brought into contacting engagement with the skin. It will be appreciated that the light bulb or heat generator 24 is of a type sufficient to raise the temperature of the flesh beneath the skin contacted by the annular pad 20, without burning the skin or flesh. The concept and the nature of the heating element for such purposes is known and understood by those skilled in the art, as generally described in prior art patent 4,944,297.

A display screen 26, for providing appropriate visual indicia of the operative status of the device 10, is provided within the housing as at 26. It is contemplated that the display 26 may constitute a light emitting diode display or other appropriate means, as would be appreciated by those skilled in the art. Also included as part and parcel of the device 10, and maintained within the housing 12 thereof, is an audio generator such as a beeper 28 or the like devised to emit audible indicia as the process performed by the device 10 is undertaken, in a manner to be discussed herein. Also interconnected with the housing 12 is an appropriate activation switch 30, which may be a push button or other appropriate switch mechanism. A similar switch 32 is provided for testing the strength of the battery 16, to assure that the battery is of sufficient strength to undertake a treatment sequence.

The housing 12 is provided with a textured hand grip area 34, or other suitable mechanism, for convenience of hand manipulation. Itwill be appreciated that the device 10 and the housing 12 are configured to be a hand sized unit, for ease of implementation, the device 10 being a hand held device.

Maintained within the housing 12, and performing the operative functions thereof, is a control unit 36, which comprises a dedicated microprocessor or chip, programmable to perform the operations to be discussed herein. The availability and programmability of such chips is well known and understood by those skilled in the art. Also included as part and parcel of the invention and as a key portion thereof, is a treatment actuator 38, removably received by the housing 12, and in physical and communicative engagement with the control unit 36 to effect the operation of the control unit 36 to an extent and by a method authorized by the treatment actuator 38.

As shown in Fig. 2, the treatment actuator 38 comprises a band or clip 40 having a registration tab 42 extending from one side thereof. The clip 40 and tab 42 are configured to be removably received by the housing 12. Carried by the clip 40 is a programmable chip 44, serving as a treatment activator, and in physical and communicative engagement with the control unit 36 by means of the connector pins shown.

As shown in Fig. 3, the control unit 36 comprises a dedicated microprocessor or chip 46, interconnected with the battery 16 and all of the operative input/output connectors of the device 10. The chip 46 contains the necessary programming to energize the light bulb or heating element 24 for a predetermined period of time upon actuation by the activation switch 30. After the heating element 24 is deactivated under control of the program of the chip 46, a quiescent period of a fixed duration is engaged, after which the program of the chip 46 causes the beeper 28 to beep, signaling the user that the activation switch 30 may again be engaged, to energize the heating element 24 for a period of time as dictated by the program of the chip 46.

The visual display 26 serves several purposes, producing an output signal indicating the treatment stage presently being entertained, also indicating the strength of the battery 16, when tested by depression of the battery test switch 32, and indicating the viability of the activator for enabling use of the device 10.

The activator 44 may be of any of various natures. It is contemplated in the first instance, that the activator may be employed to simply enable the chip 46 to run its program upon demand by the actuation switch 30. The treatment activator or programmable chip 44 may be programed to allow the chip 46 to undertake a predetermined number of treatments, and then be discardable and replaceable by a new activator chip 44 for additional subsequent treatments, if desired. In this regard, the user only pays for the treatments actually used or desired, allowing the cost of the device 10 to remain reasonable. Additionally, the activator 44 may be selected from a group of activators, each having a different program associated therewith for actuating the heat element 24. In other words, for treatment of a cold sore, a particular sequence of heat applications is desired. For an insect or spider bite, or a bee sting, a different sequence may be required. Accordingly, the activator may be selected for the treatment to be provided.

It is also contemplated that the activator 44 may be either an active or a passive circuit element. The activator 44 may itself contain a program to interface with the program of the chip 46, or the activator 44 may contain nothing more than an indicator to the chip 46 of a particular program stored by the chip 46 that must be run. The activator 44 may be a one-shot activator, capable of only activating the chip 46 a single time, or it may provide for multiple activations, to accommodate multiple treatments or uses. The activator 44 may itself include a down counter or the like, being inhibited from further use after counting down to "zero," or the actuator 44 may be disabled from further use by communication from the program of the chip 46, once the treatments authorized thereby have been employed.

With the foregoing description in mind, an appreciation may now be obtained of the use of the device 10 in the treatment of a malady, which, for purposes of discussion, will be considered to be a cold sore. In this case, the device 10 has been equipped with a treatment activator 38, with the housing 12 receiving the clip 40 and registration tab 42 thereof. Each treatment of the cold sore requires three spaced apart heat applications of particular time duration. In a preferred embodiment, the activator chip 44 of the treatment activator 38 is programmed to apply three such treatments of three separate timed and spaced apart heat applications.

To begin use, the user first depresses the battery test switch 32, causing the chip 46 to test the voltage of the battery 16, to determine if it is sufficient to undertake the treatment authorized by the treatment activator 38. Such indicia is presented upon display 26. In a preferred embodiment, the letter "H" appears on display 26 if the battery voltage is above a first threshold indicative of a strong battery, and the letter "L" appears if the battery voltage is below that first threshold, but above a second lower threshold. In either of these events, there is sufficient voltage to provide the treatment requested. The appearance of the "L" simply informs the user that the battery is at the lower end of its useful life, and will soon need to be replaced. In the event that the battery is below the second threshold, nothing appears on the display 26, indicating that the battery is insufficient to undertake the treatment, and battery replacement is required.

To begin treatment, the user dries the area about the lip to which the annular pad 20 will be applied. The pad 20 is then gently placed against the surface of the skin and the activation switch 30 is depressed. Under control of the program of the chip 46, the number "1" will begin to flash on the display 26, and the audio generator or beeper 28 will begin to beep. Once the beeping starts, the activation switch 30 can be released and the program of the chip 46 causes the beeping and the flashing of the number 1 on the display 26 to continue while the heating element 24 is energized to apply heat to the area within the annular pad 20. In the preferred embodiment of the invention, this continues for 30 seconds, at which time the beeping stops and the numeral 1 terminates its flashing. The device 10 then remains quiescent, while a timer of the chip 46 times out a particular period of time, such as 60 seconds. At the end of that time, the beeper sounds again, alerting the user that the activation switch 30 should be depressed again, with the annular pad 20 upon the treatment area. At this time, the number 2 begins to flash on the display 26, and the beeper 28 continues to beep while the heating element 24 is activated to apply heat for a second 30 second period. At the end of this second period, the beeping stops, the flashing numeral 2 terminates, and the heating element 24 become deactivated for a second quiescent period of 60 seconds. At the end of the second quiescent period, the beeper 28 sounds again, advising the user that the third and final application of heat may be applied. At this time, the user depresses the activation switch 30, which commences the flashing of the numeral 3 on the display 26, the beeping of the beeper 28, and the activation of the heating elements 24 for a third 30-second period. At the end of this third 30-second period, the treatment of the cold sore area is complete.

If the treatment activator 30 is programmed for three treatments, either the activator 44 is down counted, or the microprocessor 46 registers the fact that only two treatments remain available from the activator 44 presently in place. If, however, the treatment activator 38 and associated activator chip 44 was capable of but a single treatment, the treatment activator 38 is now spent, and must be discarded and replaced with a new treatment activator 38 for any subsequent treatments.

Thus it can be seen that the various aspects of the invention have been satisfied by the structure presented above. A device and method for the therapeutic heat treatment of localized areas of skin and flesh is provided, with the sequence of such treatment being preprogramed, with audible and visual prompts given to the user. A treatment activator, having a programmable treatment activator chip, assures that the treatment given is proper for the issue being addressed, and also allows the user to provide for treatment on a "pay as you go" basis, rendering the device and the treatments given affordable. Moreover, the use of the treatment activator 38 allows for adaptability of the device 10 for treatment of maladies apart from cold sores, but for other issues for which the localized application of heat is beneficial.

In the example given, the cold sore treatment was presented as three 30-second applications spaced 60 seconds apart. It will be appreciated that such treatment may vary as to duration and spacing, it being contemplated that the heat application durations may be on the order of 25-45 seconds, with the spacing commensurately being on the order of 45-90 seconds.

Thus it can be seen that the aspects of the invention have been attained by the structure and technique presented above. While in accordance with the patent statutes only the best mode and preferred embodiment of the invention has been presented and described in detail, the invention is not limited thereto or thereby. Accordingly, for an appreciation of the true scope and breadth of the invention reference should be made to the following claims.

## Claims

1. A therapeutic treatment device, comprising:
a housing configured to be hand held;
a pad on said housing configured to engage an area on the body of a user;
a heat source adjacent said pad; and
a controller connected to said heat source;
a removable and replaceable actuator module in interconnection with said controller, said actuator module enabling said controller to enable said heat source for said predetermined periods of time at said predetermined intervals, wherein said predetermined periods of time at predetermined intervals is a function of a particular malady being treated.

2. The therapeutic treatment device according to claim 1, further comprising a signal generator connected to said controller, said signal generator producing a first detectable signal at least at certain of said predetermined intervals.

3. The therapeutic treatment device according to claim 2, wherein said signal generator further produces a second detectable signal during at least certain of said predetermined periods of time.

4. The therapeutic treatment device according to claim 1, further comprising a visual display connected to said controller, said visual display producing visual indicia of the state of enablement of said heat source.

5. The therapeutic treatment device according to claim 1, wherein said actuator module is removably received by said housing.

6. The therapeutic treatment device according to claim 1, wherein said actuator module is **characterized by** a predetermined number of cycles of said enabling said controller to enable said heat source for said predetermined periods of time at said predetermined intervals.

7. The therapeutic treatment device according to claim 6, wherein said actuator module is inhibited from enabling said controller following said predetermined number of cycles.

8. The therapeutic treatment device according to claim 7, wherein said actuator module is an active module, having a counter for monitoring a number of cycles of said enabling said controller.

9. An actuator for a therapeutic treatment device having a housing containing a heat source and controller connected to said heat source, said controller enabling said heat source for predetermined periods of time at predetermined intervals, the actuator comprising:
a clip configured for removable receipt by the housing; and
a module carried by said clip, said module configured for communicative interconnection with the controller, wherein said module is selected from a group of modules having different characteristic predetermined periods of time at predetermined intervals.

10. The actuator for a therapeutic treatment device according to claim 9, wherein said module is **characterized by** a predetermined number of cycles of enabling said heat source for predetermined periods of time at predetermined intervals said predetermined periods of time and said predetermined intervals being associated with a specific treatment intended by said therapeutic treatment device.
